# EUROPEAN PATENT APPLICATION

(11) **EP 2 143 454 A1**
(43) Date of publication of application: **13.01.2010**
(21) Application number: 08104654.2
(22) Date of filing: 07.07.2008
(51) Int. Cl.: A61M 5/315, A61M 5/19, A61J 1/20

(54) **Dispensing device, kit containing the device, and method of operating the device**

(71) Applicant: Kuros Biosurgery AG, 8005 Zürich (CH)
(72) Inventor: Lutz, Peter, 8455 Rüdlingen (CH); Rehor, Annemie, 8400 Winterthur (CH)
(74) Representative: Hepp, Dieter

(57) **Abstract**

The application relates to a dispensing device (1) containing at least one chamber (5,5') for receiving a fluid (B,B'), a plunger unit (6) comprising at least one piston (7,7'), locking means and counter-locking means as well as catching means and counter-catching means. The locking means and the counter-locking means can be brought into a locking position (L), in which a movement of the plunger unit (6) in one or both directions is substantially prevented. The catching means and the counter-catching means can be brought into an engagement position (E), in which the locking means and the counter-locking means cannot be brought into the locking position (L). Furthermore, the application relates to a kit (19) containing the dispensing device (1) and to a method of operating the device (1) or kit (19).

## Description

The present invention relates to a dispensing device, a kit containing the device, and a method of operating the device. Such a device may be used for receiving, storing, and ejecting at least one fluid, in particular several fluids. In some embodiments, the device may be used for ejecting several fluids to be mixed directly upon ejection.

A comparable dispensing device in the form of a syringe is shown in DE 1 053 143. This device has a main body with a front end, an opposite rear end, and a chamber between the front end and the rear end for receiving a fluid. Furthermore, the device contains a plunger comprising a piston, which is arranged slidingly in the chamber.

The chamber can be filled with an injection fluid by directly or indirectly connecting it with a fluid reservoir and retracting the plunger. The plunger contains several locking teeth which can be locked relative to the main body with the help of a locking lever. With these locking means and counter-locking means, a locking position may be obtained, in which a movement of the plunger in the direction from the rear end to the front end is prevented, thereby also preventing the ejection of the fluid. In order to release this locking, the locking lever is activated, which releases the plunger again and thereby allows the ejection of the fluid.

However, the device disclosed in this document has several disadvantages. Inter alia, the operation of this device is rather complicated. In particular, the locking lever has to be activated for the entire period of time during which the plunger is to be moved.

Moreover, EP 420 126 discloses a blood sample removal device comprising locking teeth which are engageable with a resilient locking stop, which prevents a movement of the piston in one direction. However, the engagement of the locking teeth and the locking stop cannot be reversed during a proper operation of the device. Consequently, the plunger cannot be re-inserted into the chamber, thereby preventing a re-ejection of the fluid through the inlet opening.

It is therefore an object of the present invention to provide a dispensing device which overcomes the drawbacks of the prior art, which in particular allows for an easy operation.

This goal is achieved by a dispensing device containing at least one chamber, a plunger unit, locking means, and counter locking means, wherein, according to the invention, the device further comprises catching means and counter-catching means.

The dispensing device may contain only a single chamber such as the device described in DE 1 053 143. However, preferably, the device is a multi-chamber dispensing device or, more preferably, a two-chamber dispensing device. Such a two-chambered dispensing device is known, for example, from WO 2007/131371.

The dispensing device contains a main body comprising a front end and an opposite rear end. Between the front end and the rear end, there is arranged at least one chamber for receiving a fluid. Each of the chambers comprises an opening at the front end of the main body, which serves as a fluid inlet and/or outlet. The fluid may be a gas. However, preferably, the fluid to be received is a liquid. The viscosity of the liquid can vary over a wide range, i. e. from paste-like to liquid.

The plunger unit comprises at least one piston, wherein each piston is arranged slidingly in one of the chambers. Preferably, the pistons are rigidly connected to each other.

The locking means and the counter-locking means can be brought into a locking position. In the locking position, a movement of the plunger unit in one or both directions is substantially prevented. Thereby, a relative fixation of the plunger unit with respect to the main body is achieved, so that the injection of a fluid into the chamber or chambers and/or the ejection of a fluid out of the chamber or chambers is prevented. Such a fixation can be desirable when at least one of the chambers is subjected to an over-pressure or an under-pressure.

According to the invention, the catching means and the counter-catching means can be brought into an engagement position, in which the locking means and the counter-locking means cannot be brought into the locking position.

Therefore, when the catching means and the counter-catching means are in the engagement position, the engagement of the catching means and the counter-catching means releases the locking induced by the locking means and the counter-locking means. Thus, in the engagement position, the relative movement of the plunger unit with respect to the main body is not restricted in one or both directions. Consequently, the relative movability of the plunger unit with respect to the main body can be controlled by the position of the catching means and the counter-catching means.

Preferably, the locking means and the counter-locking means can not only be brought into a locking position, but also into a releasing position. In this releasing position, a movement of the plunger unit is possible in both directions. Even more preferably, the catching means and the counter-catching can be designed such that when they are in the engagement position, the locking means and the counter-locking means can be brought into the releasing position.

Therefore, when the catching means and the counter-catching means are in the engagement position, the plunger unit can be moved in both directions, which allows both an injection and an ejection of fluids by a respective movement of the plunger.

Preferably, the catching means and the counter-catching means may not only obtain an engagement position, but also a disengagement position in which the locking means and the counter-locking means can be brought into the locking position.

Consequently, when the catching means and the counter-catching means are in the disengagement position, the locking position may be obtained, in which the movement of the plunger unit in one or both directions is substantially prevented. Thus, the injection and/or the ejection of fluids are prevented.

In a preferred embodiment, in the locking position, a movement of the plunger unit in the direction from the rear end to the front end is prevented. Thus, when this locking position is obtained, a movement of the plunger is prevented when at least one of the chambers is subject to an under-pressure so that the ejection of a fluid or fluids out of the chambers is prevented. Such an under-pressure in a chamber can occur when fluids are inserted from a fluid reservoir having under-pressure.

Preferably, the catching means are different from the locking means and/or the counter-catching means are different from the counter-locking means. Thus, the mechanisms provided by the locking means and the counter-locking means on the one hand and by the catching means and the counter-catching means on the other hand may be different from one another.

In summary, the relative position of the catching means and the counter-catching means determines whether the locking means and the counter-locking means can be brought into the locking position or not, i. e. whether the movement of the plunger unit can be prevented in one direction or not. Therefore, when the catching means and the counter-catching means are in the engagement position, the plunger unit cannot be locked relative to the main body of the device unintentionally, which facilitates the injection and/or ejection of the fluid or fluids.

In a preferred embodiment, the device may contain stopping means which are arranged such that a movement of the plunger unit is limited in the direction from the front end to the rear end of the device. In any case, the stopping means do not prevent the achievement of the locking position. Therefore, the plunger unit may be retracted from the main body to such an extent that the locking position is obtained. Preferably, the stopping means prevent the complete removal of the plunger unit from the main body of the device.

According to a preferred embodiment, the stopping means are arranged such that when the locking means and the counter-locking means are in the locking position, the plunger unit is still movable in the direction from the front end to the rear end by a pre-defined distance.

Due to this construction, a fluid or fluids may be inserted from a fluid reservoir or fluid reservoirs into the chamber or chambers of the device by retracting the plunger unit until the locking position is obtained. From this locking position, the plunger unit can be further retracted from the main body by the pre-defined distance, whereby an excess of fluid or fluids is temporarily inserted into the chamber or chambers. This ensures that the openings of the device and, possibly, ducts of a connecting adapter connecting the openings and the reservoirs are completely flooded with the fluids. The plunger unit is then released again, so that it moves back to the locking position due to the under-pressure.

Preferably, the stopping means contain a first stopping surface and a second stopping surface. In one embodiment, the first stopping surface is arranged on the plunger unit, whereas the second stopping surface is arranged on the main body, in particular on the inside of one of the chambers of the device.

According to a preferred embodiment, the locking means and the counter-locking means are arranged in such a way that in the disengagement position, the following two states may be obtained: In the releasing position, the locking elements and/or the counter-locking elements are resiliently supported by a tensioned supporting element, in particular radially. In the locking position, the supporting element is less tensioned.

Thus, in the disengagement position, the locking position may be obtained from the releasing position by a release of tension of the supporting element, wherein this release of tension may occur automatically during retraction of the plunger unit. By this release, the locking means and/or the counter-locking means can move automatically, in particular radially, so that the locking position is obtained.

Preferably, the locking means comprise at least one radial protrusion, in particular at least one radially extending tooth. In one embodiment, the protrusion, in particular the tooth, is engageable with counter-locking means arranged on the main body of the device.

According to one embodiment, the plunger unit contains the catching means and the counter-catching means. However, it is also conceivable that the main body of the device contains the catching means and/or the counter-catching means.

In a preferred embodiment, the plunger unit comprises the supporting element. The supporting element may be designed as at least one retaining arm. With preference, the retaining arm is flexible and bendable in a radial direction, in particular in a radial direction. The retaining arm may further contain a tip forming the catching means and a retaining surface forming the counter-catching means. Preferably, the plunger unit, in particular the retaining arm, contains the locking means, as for example a tooth, which may be extending radially outwardly.

In one embodiment, the retaining arm extends in a longitudinal axis of the device and is fixed to a main portion of the plunger unit at one its ends which points in the direction of the rear end of the device. In this embodiment, the tip points in the direction of the front end.

Preferably, the catching means and the counter-catching means can be brought from the disengagement position into the engagement position by exerting an inward radial force on the plunger unit, in particular on the retaining arm. When the engagement position is obtained, the catching means and the counter-catching means remain in this position even when the force is removed, which simplifies the operation of the device significantly.

In one embodiment, a cavity is formed radially between the retaining arm and a main portion of the plunger unit. This cavity can receive the retaining arm in a bent position.

In certain embodiments, the retaining arm may lose its tension due to a fatigue of material when it has been maintained in a bent position over a certain time, for example during storage of the device. Therefore, the cavity between the retaining arm and the main portion of the plunger unit may be partly or completely filled with an additional elastic supporting element. The additional elastic supporting element can maintain an additional tension and provide an additional radial support of the retaining arm and the protrusion.

During filling the device with one or more fluids from one or more fluid reservoirs, air may be unintentionally entrapped in the one or more chamber of the device. This may occur, for example, when the fluids are injected via an adapter having ducts which are initially filled with air. In order to eject this unintentionally entrapped air, the device is usually held in an at least approximately vertical position, and the plunger unit is at least partially re-inserted into the chambers until the air has left the chambers through the openings.

However, when the device is not held exactly vertical, the air entrapments in only some of the chambers are removed, or only parts of the air entrapments are removed. In those chambers in which, due to the tilting of the device, the air entrapment is not adjacent to the opening in this chamber, only fluid is ejected, but not the air. This problem may occur, for example, with the device shown in WO 2007/131371.

The document US 4,040,420 discloses a two-chamber device comprising a tapered nozzle and an off-set conical transition section joining the nozzle to the chambers. A similar device is disclosed in DE 20 2006 005 663. The shapes of the chambers shown in these two documents facilitate the removal of air entrapments occurring during an unintentional tilting. However, the devices shown there are disadvantageous since the chambers cannot be completely emptied, which means a waste of fluid. Moreover, already before filling the chambers with the fluids, there are air entrapments in these chambers.

Therefore, according to another and independent aspect of the present invention, a tip surface of at least one piston is essentially complementary to an end surface of the respective chamber which contains the opening. Consequently, prior to injecting the fluids into the chambers, when the pistons are completely inserted, there are essentially no air entrapments in the chambers. Furthermore, during re-ejection of the fluids by re-inserting the pistons, essentially no fluids can remain in the chambers.

According to the invention, at least one of the chambers, in particular the end surface of at least one of the chambers, is designed such that its cross-sections is reduced in the direction of a respective opening, i. e. in the direction of the front end. The device exhibits an (imaginary) tilting axis which is perpendicular to a longitudinal axis of the device. The longitudinal axis of the device may be tilted from the vertical direction about this tilting axis in two opposed (rotational) tilting directions. According to the invention, the device exhibits a critical tilting angle such that the following property is satisfied for a tilting in at least one of the two tilting directions: When the device is tilted by a tilting angle not exceeding a critical tilting angle, all chambers have essentially no dead volume. A chamber has "no dead volume" for a specific tilting angle when no air entrapment can be formed which is not adjacent to the opening in the end surface. In other words, when the chamber has no dead volume for a specific tilting angle, all possible air entrapments are situated adjacent to this opening and can therefore be removed by moving the piston arranged in this chamber.

However, when the tilting angle exceeds the critical tilting angle and is maintained during the ejection of the fluid, the air entrapment in the chamber can only escape through the opening when the piston is completely inserted into the chamber, i. e. when its tip surface contacts the end surface of the chamber. Therefore, the entrapped air can only escape when the entire fluid has already been ejected through the opening.

In preferred embodiments, the critical tilting angle is at least 10°, more preferably at least 15°, even more preferably at least 20°, most preferably about 25°.

Furthermore, according to the invention, the openings are arranged adjacent to one another. Particularly, the openings may have a distance from each other which is smaller than one-half of the sum of the inner dimensions of the chambers in a plane perpendicular to their longitudinal axis. This embodiment allows the ejection of fluids from nearby positions and can therefore facilitate an optional subsequent mixing of the fluids, thereby diminishing the necessary size of a mixing assembly to be used.

In one embodiment, the end surface of at least one chamber is essentially planar. Preferably, the end surfaces of all chambers are essentially planar. At least one of the planar end surfaces is inclined by an angle with respect to a plane which is perpendicular to a longitudinal axis of the device. The surfaces are inclined such that when the device is in an upright position, the opening is adjacent to the topmost portion of the surface. Here, the device is in an upright position when its longitudinal axis is vertical and the front end is directed upwards. Preferably, this angle is at least 10°, more preferably at least 15°, even more preferably at least 20°, most preferably about 25°. In these embodiments, this angle corresponds to the critical tilting angle of the device.

It is conceivable that the device exhibits several tilting directions, such that for any tilting angle in any of these tilting directions, which does exceed the critical tilting angle, all chambers have essentially no dead volume. For example, the device may be tiltable about a tilting axis in both opposite tilting directions.

A further aspect of the invention is directed to a multiple connecting adapter, in particular a double connecting adapter. Such a multiple connecting adapter can be used for indirectly connecting each of at least two openings of a multi-chamber dispensing device with a respective fluid reservoir simultaneously. Moreover, it can be used for simultaneously transferring fluids contained in the fluid reservoirs to respective chambers of the device via the openings. The multi-chamber dispensing device may have some or all of the properties described above.

According to the invention, the multiple connecting adapter comprises at least two ducts. A first end of each duct opens out into a respective device-facing opening, which is connectable or connected with a respective opening of the device. Furthermore, a second end of each duct, which is opposite to the respective first end of the duct, opens out into a respective reservoir-facing opening, which is connectable or connected with a respective fluid reservoir.

A further aspect of the invention is concerned with a kit containing a device according to the present invention as well as at least one of the following components:
The kit may contain a multiple connecting adapter, in particular a multiple connecting adapter as described above.
Furthermore, the kit may contain a mixing assembly for mixing fluids ejected from the chambers. Additionally, the kit may contain a spray assembly for spraying fluids to be ejected from the chambers. Such mixing assemblies and spray assemblies are described, by way of example, in WO 2007/131371.

A further aspect of the invention relates to a method of operating a device or a kit containing the device according to the invention. The method comprises the following steps:
i) providing at least one fluid reservoir, at least one of the fluid reservoirs containing a fluid;
ii) directly or indirectly connecting openings of each chamber of the device with a respective fluid reservoir, in particular by a multiple connecting adapter;
iii) at least partially retracting the piston or pistons of the device from the chamber or chambers;
iv) allowing the locking means and the counter-locking means to obtain the locking position;
v) disconnecting the openings from the respective fluid reservoirs;
vi) bringing the catching means and the counter-catching means into the disengagement position, in particular by a one-hand operation;
vii) at least partially inserting the pistons into the chambers, thereby ejecting the fluids, in particular through a mixing assembly and/or a spray assembly.

Preferably, prior to step i), the device is provided in a state in which the pistons are essentially completely inserted in the chambers. Alternatively, the device may be brought into this state.

The invention will now be explained in more detail by nonlimiting examples and figures, wherein
- Figure 1: shows a dispensing device according to the present invention in the simultaneous releasing position and disengagement position;
- Figure 2: shows the dispensing device of Figure 1 in the simultaneous locking position and disengagement position;
- Figure 3: shows the dispensing device of Figure 1 in the simultaneous locking position and disengagement position, wherein the first stopping surface and the second stopping surface are in contact;
- Figure 4: shows the dispensing device of Figure 1 in the simultaneous releasing position and engagement position;
- Figures 5a to 5c: show a portion of the dispensing device of Figure 1 at different tilting angles with filled-in fluids and air entrapments;
- Figure 6: shows a kit according to the invention containing a dispensing device and a connecting adapter;
- Figure 7: show a kit according to the invention containing a dispensing device, a mixing device, and a spraying device.

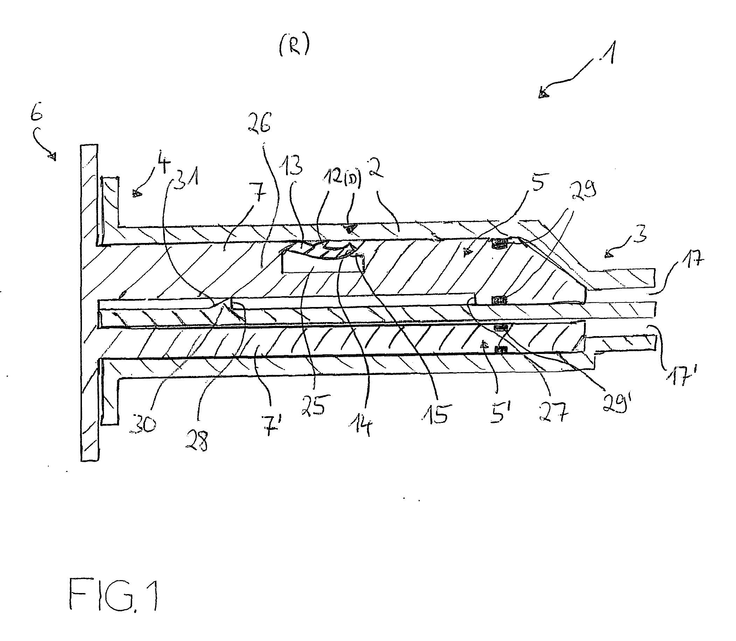

The dispensing device 1 depicted in Figures 1 to 4 has a main body 2 comprising a front end 3 and an opposite rear end 4. The main body 2 comprises two chambers 5,5' between the front end 3 and the rear end 4 for receiving a fluid. The chambers 5,5' have a length of 85 mm. Both chambers 5,5' have a circular cross section, wherein the cross section of the first chamber 5 is larger than the cross section of the second chamber 5'. The ratio of the cross section may be, for example, 1:1, 4:1 or 10:1. In particular, the cross sections may be 10/11 cm² and 1/11 cm². It is to be noted that the figures are not drawn to scale. Each of the chambers 5,5' has an opening 17,17' at the front end 3, which serves as a fluid inlet and/or outlet. The device 1 further comprises a plunger unit 6 comprising two pistons 7,7', each of which is arranged slidingly in one of the chambers 5,5'. Both pistons 7,7' are rigidly connected to each other. In particular, the entire plunger unit 6 including the pistons 7,7' may be an integral part. Each of the pistons 7,7' carries a respective o-ring 29,29', which serve to seal the chambers 5,5'.

The plunger unit 6 comprises a retaining arm 13, which extends in the longitudinal axis of the device 1 and is fixed to a main portion 26 of the plunger unit 6 at its end pointing in the direction of the rear end 4. The retaining arm 13 contains a tip 14 pointing in the direction of the front end 3. The tip 14 forms catching means according to the invention. Radially between the retaining arm 13 and the main portion 26 of the plunger unit 6, a cavity 25 is formed. Furthermore, the plunger unit 6 comprises a retaining surface 15, which forms counter-catching means. Additionally, the retaining arm 13 contains a protrusion in the form of a radially extending tooth 12, which forms locking means according to the invention.

Moreover, the plunger unit 6 contains a first stopping surface 27, and the first chamber 5 contains a second stopping surface 28. The first stopping surface 27 is formed as a portion of the surface of a cam 30. The opposite surface 31 of the cam 30 is chamfered in order to allow the assembly of the device 1. The first stopping surface 27 and the second stopping surface 28 form stopping means which limit the movement of the plunger unit 6 in the direction from the front end 3 to the rear end 4 of the device 1. In particular, the first stopping surface 27 and the second stopping surface 28 are arranged such as to prevent the complete removal of the plunger unit 6 from the main body 2 of the device 1. It is obvious that the device 1 may contain more than one cam 30 containing first stopping surfaces 27. Moreover, the cam or cams 30 may be arranged at any angle around the longitudinal axis A of the device 1. In particular, at least one of the cams 30 may be arranged outside the plane defined by the longitudinal axis A and the retaining arm 13, i. e. outside the drawing plane in Figures 1 to 4.

The main body 2 and/or the plunger unit 6 comprising the pistons 7,7' are composed of or contain a plastic material. They can be manufactured, for example, by injection molding. In one embodiment, the plunger unit 6 containing the pistons 7,7' and the retaining arm 14 may be injection-molded as an integral part. In a particular embodiment, the plunger unit 6 may be made from a fiber-glass reinforced plastic material.

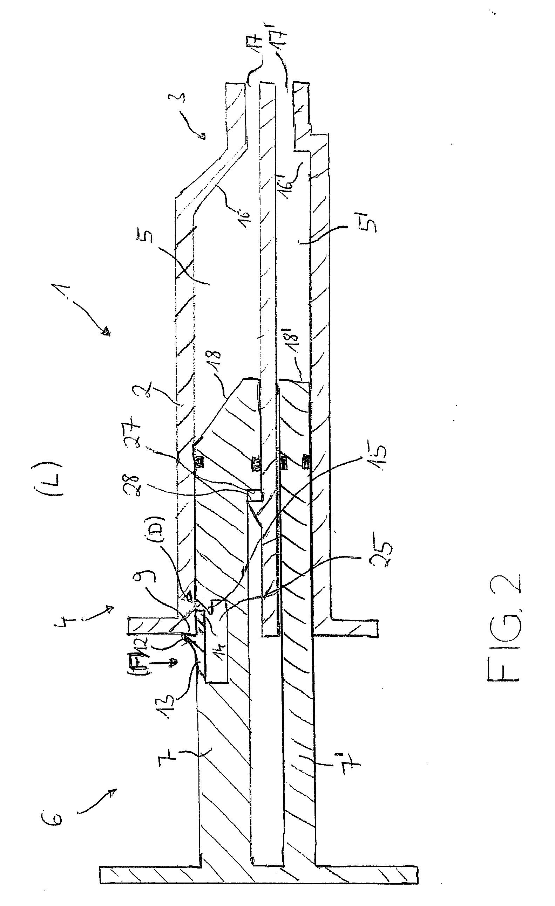

In the state shown in Figure 1, the tip 14 of the retaining arm 13 is situated on the radially outwardly directed side of the retaining surface 15. Hence, the tooth 12 arranged on the retaining arm 13 is in contact with the inner surface of the first chamber 5. Due to the contact of the tooth 12 with the inner wall of the first chamber 5, the retaining arm 13 is bent. Accordingly, the tooth 12 is resiliently radially supported against the inner surface of the first chamber 5. The tip 14 and the retaining surface 15 are in a disengagement position D, which allows a locking position (cp. Figure 2 and the corresponding description below). However, the contact between the tooth 12 and the inner surface of the first chamber 5 does not significantly prevent the movement of the plunger unit 6 in both directions, so that, in Figure 1, the releasing position R is present.

When the plunger unit 6 is retracted from the main body 2 to a certain extent, the tooth 12 snaps behind a rear surface 9 of the main body 2, whereby the rear surface 9 forms counter-locking means. The outward radial movement of the tooth 12 is automatically initiated by a release of tension of the retaining arm 13. Thus, the retaining arm 13 functions as supporting element. In the state shown in Figure 2, the tooth 12 and the rear surface 9 are engaged in a locking position L, in which a movement of the plunger unit 6 in the direction from the rear end 4 to the front end 3 is prevented. Thus, even when an under-pressure is present in one of the chambers 5,5', the pistons 7,7' cannot be pulled back into the chambers 5,5'. As can be seen from Figure 2, the arrangement of the first stopping surface 27 and the second stopping surface 28 do not prevent the achievement of the locking position L.

In certain embodiments, the retaining arm 13 may lose its tension due to a fatigue of material when it has been maintained in the bent position as shown in Figure 1. This fatigue may prevent the movement to the locking position L shown in Figure 2. Therefore, the cavity 25 may be partly or completely filled with an additional elastic supporting element, which is not shown in the drawings. The additional elastic supporting element can maintain an additional tension, thereby eliminating the above-mentioned problem of fatigue. Preferably, the additional elastic supporting element is an elastic pad, such as a silicone pad, or a spring.

The disengagement position D of the tip 14 and the retaining surface 15 is still maintained, even when the locking position L is obtained as in Figure 2.

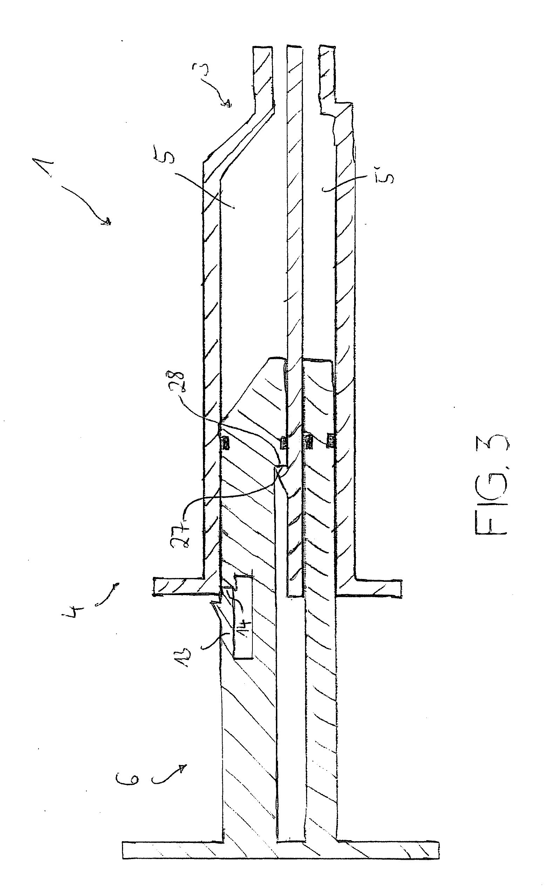

From the locking position (L) according to Figure 2, the plunger unit 6 can be further moved in the direction from the front end 3 to the rear end 4 until the first stopping surface 27 and the second stopping surface 28 are in contact, so that the position shown in Figure 3 is obtained. The arrangement of the first stopping surface 27 and the second stopping surface 28 determines the distance by which the plunger unit 6 is further retractable.

Furthermore, the retaining arm 13 is dimensioned and arranged relative to the first stopping surface 27 and the second stopping surface 28 such that its tip 14 does not leave the first chamber 5, which facilitates the insertion of the plunger unit 6 to be performed in a later step (see below).

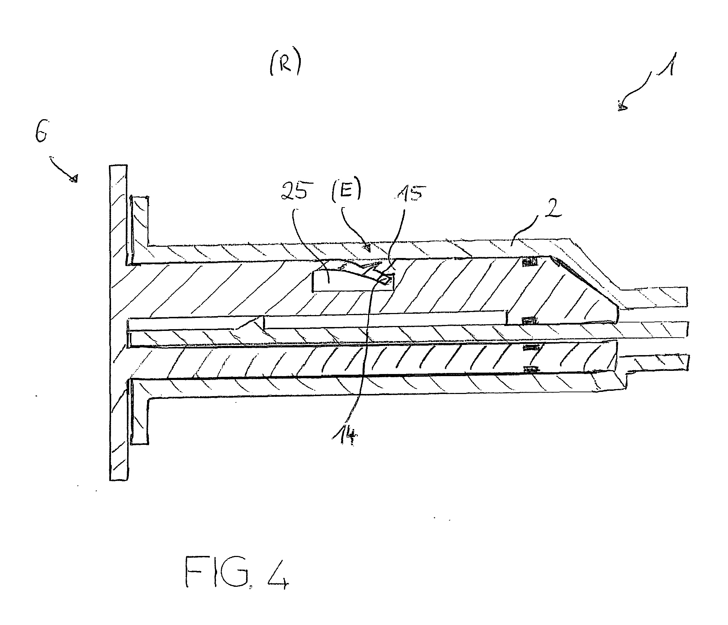

By exerting an inward radial force F on the retaining arm 13, the latter is pushed radially inwardly and further into the cavity 25. As a result, the tip 14 snaps behind the retaining surface 15, whereby an engagement position E is obtained between the tip 14 and the retaining surface 15 (cp. Figure 4). This force F may be applied with a single finger, which significantly facilitates the operation.

Once the engagement position E is obtained, the tooth 12 cannot contact the rear surface 9 of the main body 2 and the locking position cannot be obtained anymore. Therefore, even when the operator's finger is removed and, thereby, the force F is also removed, the engagement position is maintained, which further simplifies the operation.

In the engagement position E thus obtained, the plunger unit 6 is moveable again in both directions (with the limitation induced by the first stopping surface 27 and the second stopping surface 28), so that the releasing position R is obtained again and maintained. In this releasing position R, the plunger unit 6 may be re-inserted into the main body 2 again, so that the state shown in Figure 4 can be obtained. In this state depicted in Figure 4, the tooth 12 is not in contact with the inner surface of the first chamber 5.

Concludingly, in the engagement position E, the tooth 12 forming the locking means and the rear surface 9 forming the counter-locking means cannot be brought into the locking position L, since the tip 14 of the retaining arm 13 forming the catching means and the retaining surface 15 forming the counter-catching means are engaged with each other.

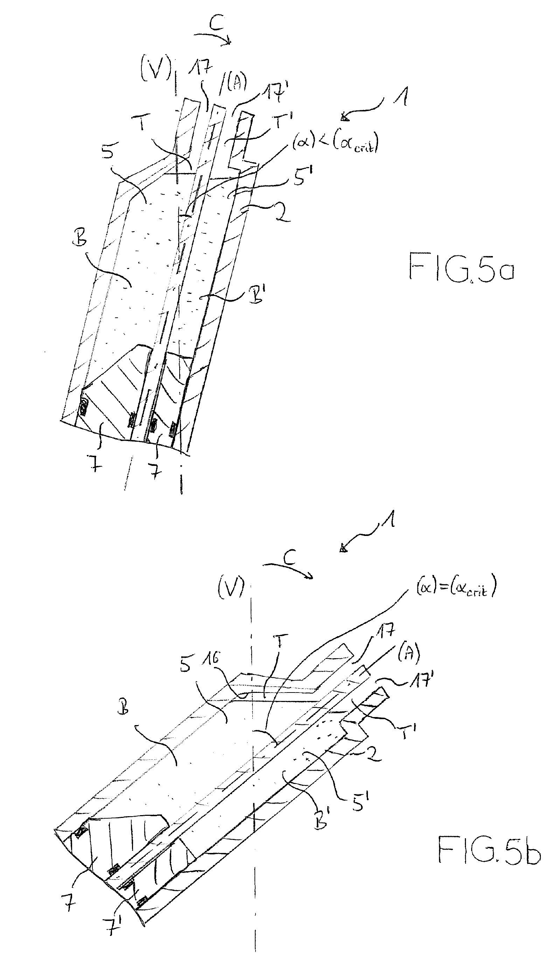

As shown in Figures 1 through 4 and also in Figures 5a to 5c, the end surface 16 of the first chamber 5 is inclined from a plane which is perpendicular to the longitudinal axis A of the device 1. Hence, the cross-section of the first chamber 5 is reduced in the direction of the opening 17, i. e. in the direction of the front end 3. The end surface 16' of the second chamber 5' is perpendicular to the longitudinal axis A. Both end surfaces 16,16' are essentially planar and are adjacent to the openings 17,17'. The openings 17,17' are arranged adjacent to one another, so that the distance between the openings 17,17' is smaller than one-half of the sum of the inner dimensions of the chambers 5,5' in the drawing plane. In particular embodiments, the distance between the centers of the openings may be 3.2 mm or 4 mm. This allows the ejection of fluids at positions which are close to each other and can therefore facilitate an optional subsequent mixing of the fluids, in particular by a relatively small mixing assembly.

The tip surfaces 18 of the pistons 7,7' are complementary to the end surfaces 16 of the respective chambers 5,5'. Therefore, prior to injecting fluids into the chambers 5,5', when the pistons 7,7' are completely inserted, there are essentially no air entrapments in the chambers 5,5'. Furthermore, during re-ejection of fluids through the openings 17,17' by re-inserting the pistons 7,7', essentially no fluid can remain in the chambers 5,5'.

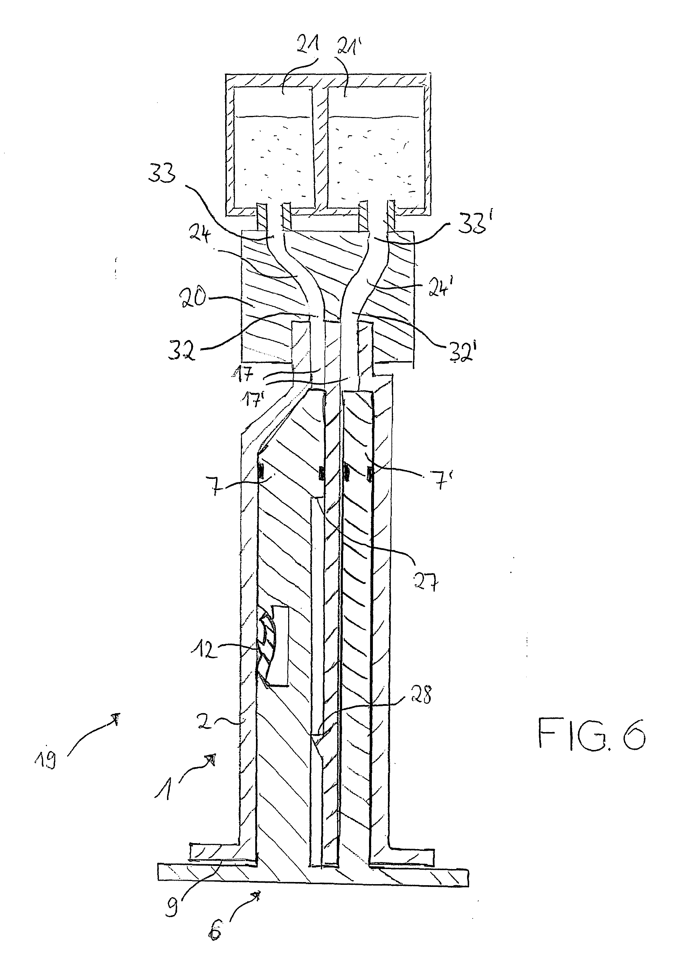

The purpose of the inclination of the end surface 16 is illustrated in Figures 5a to 5c. Each of the two chambers 5,5' contains a liquid B,B' and an air entrapment T,T'. The air entrapments T,T' may originate from air enclosures in a connecting adapter (cp. Fig. 6 and the description relating thereto). In Figure 5a, the longitudinal axis A of the device 1 is tilted from the vertical axis V in the tilting direction C about a tilting axis by an angle α. The tilting axis is perpendicular to the drawing plane. In Figure 5a, the tilting angle α does not exceed a critical tilting angle α_{crit}, which is approximately 50°. For this tilting angle α shown in Figure 5a, the air entrapments T,T' are adjacent to the openings 17,17' in the end surfaces 16,16'. Therefore, by inserting the plunger unit (of which only the pistons 7,7' are shown in Figures 5a and 5b), the air entrapments T,T' are ejected through the openings 17,17' simultaneously, leaving no dead volume of air entrapments.

In Figure 5b, the longitudinal axis A is tilted from the vertical axis V in the tilting direction C by an angle α which is equal to the critical tilting angle α_{crit}. For this tilting angle α, the surface of the fluid B in the first chamber 5 is parallel to the end surface 16 of this first chamber 5. Thus, this is the maximum tilting angle for which the air entrapment T is adjacent to the opening 17 and can therefore be ejected through the opening 17 by inserting the plunger unit 6.

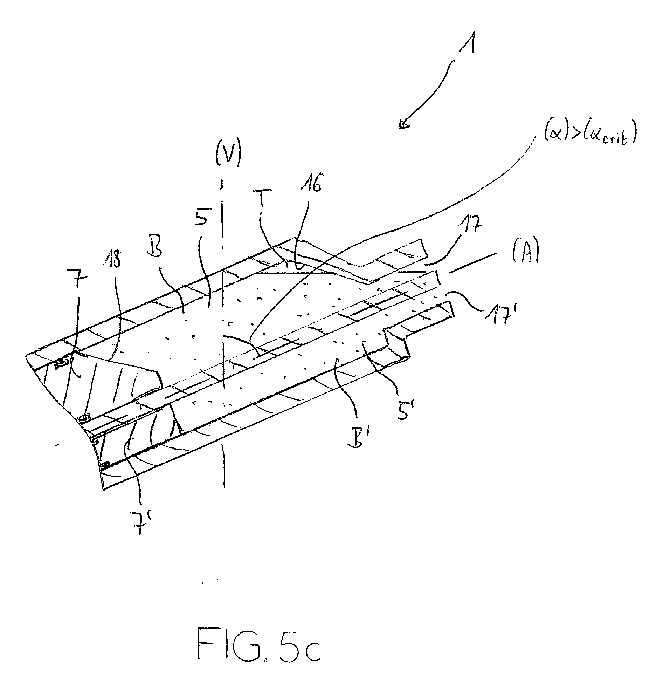

Finally, Figure 5c depicts a situation in which the tilting angle α exceeds the critical tilting angle α_{crit}. With this tilting angle α, the air entrapment T in the first chamber 5' can only escape through the opening 17 when the piston 7 is completely inserted into the chamber 5, so that its tip surface 18 contacts the end surface 16. However, in this position, the entire fluid B has already been ejected through the opening 17.

The specific embodiment disclosed above exhibits catching means and counter-catching means as well as corresponding surfaces of the piston tips and chambers, along with the respective advantages described above. However, it is clear that both aspects are independent from one another and that the omission of one of the aspects does not impede the function and advantages of the other aspect. For example, the device may exhibit catching means and counter-catching means according to the invention but have chambers with plane end surfaces which are perpendicular to the longitudinal axis of the device.

Figure 6 shows a kit 19 containing a dispensing device 1, a connecting adapter 20, and two fluid reservoirs 21,21'. For simplicity, only a part of the device is shown. The connecting adapter 20 contains two ducts 24,24', each of which connects one of the openings 17,17' of the device 1 with a respective fluid reservoir 21,21'. More explicitly, a first end of each duct 24,24' opens out into a respective device-facing opening 32,32', which is connectable or connected with a respective opening 17,17' of the device 1. Furthermore, a second end of each duct 24,24', opposite to the respective first end of the duct 24,24', opens out into a respective reservoir-facing opening 33,33' which is connectable or connected with a respective fluid reservoir 21,21'. The connecting adapter 20 thereby allows a simultaneous filling of the first chamber 5 and the second chamber 5'.

According to the method of the invention, the pistons 7,7' are retracted from the respective chambers 5,5', in particular by retracting the plunger unit 6 from the main body 2. During this retraction, air which was initially contained in the openings 17,17' and/or the connecting adapter 20 enters the chambers 5,5', which leads to air entrapments as shown in Figures 5a to 5c. By an optional reciprocating motion of the plunger unit 6, these air entrapments can be successively moved out of the chambers 5,5', through the openings 17,17' and the ducts 24,24' and into the reservoirs 21,21'.

When the fluid reservoirs 21,21' and the adapter 20 are sealed from the environment, this retraction has to be performed against a force induced by an under-pressure. The retraction is performed at least until the tooth 12 and the rear surface 9 of the main body 2 enter the locking position L shown in Figure 2. In this locking position L, the plunger unit 6 cannot be pulled back into the main body 2 by the under-pressure.

Preferably, the plunger unit 6 is then further retracted from the main body 2 until the first stopping surface 27 and the second stopping surface 28 come into contact, as shown in Figure 3. Thus, an excess of fluids B,B' is temporarily inserted into the chambers 5,5', which ensures that the openings 17,17' of the device 1 and the ducts 24,24' of the connecting adapter 20 are completely flooded with the respective fluids B,B'. The distance covered by the plunger unit 6 during this further retraction is pre-defined by the arrangement of the first stopping surface 27 and the second stopping surface 28.

According to a preferred embodiment, the first stopping surface 27 and the second stopping surface 28 are arranged such that the excess volume temporarily inserted in the chambers 5,5' is about 0.3 ml. This value has shown to be an advantageous compromise which guarantees that, on the one hand, the openings 17,17' and the ducts 24,24' are completely flooded with the respective fluids B,B' and, on the other hand, the under-pressure is not increased to such an extent that environmental air can enter the system through possible leaks.

In a next stop, the plunger unit 6 is released again, so that it moves back to the locking position due to the under-pressure which is present in the chambers 5,5'.

Subsequently, the openings 17,17' are disconnected from the respective fluid reservoirs 21,21' by removing the connecting adapter 20 from the openings 17,17'. Due to the remaining under-pressure in the fluid reservoirs 21,21', the fluids B,B' within the ducts 24,24' of the connecting adapter 20 will be sucked back into the respective fluid reservoirs 21,21'. Therefore, the fluids B,B' cannot drop out of the connecting adapter 20 during or after the process of disconnecting. This prevents an unintentional mixing and, possibly, a curing of the fluids B,B', which could lead to a clogging of the openings 17,17'.

In the next step (which is not illustrated), the tip 14 of the retaining arm 13 is pushed behind the retaining surface 15 by exerting a force on the retaining arm 13. This operation may be performed with a single finger, which simplifies the process. Thus, the engagement position E is obtained, in which the locking position is disabled, so that the plunger unit 6 is again movable in both directions, as was described above in relation to Figures 2 to 4. When the force is removed, the engagement position is maintained, so that a return to the locking position is further impeded. Since at this time, an under-pressure is not present in the chambers 5,5' anymore, the pistons 7,7' cannot be pulled back into the chambers 5,5' unintentionally.

In a next (optional) step, possible air entrapments which might still be contained in the chambers 5,5' are ejected, as was described above in relation to Figures 5a and 5b.

In a further (optional) step, a mixing assembly 22 and a spray assembly 23 are connected to the openings 17,17'. However, it is also conceivable that the openings 17,17' are already disconnected from the multiple connecting adapter 20 and the mixing assembly 22 and the spray 23 assembly are connected to the openings 17,17' before the disengagement position is obtained, i. e. before the tip 14 of the retaining arm 13 is pushed behind the retaining surface 15 by exerting a force on the retaining arm 13.

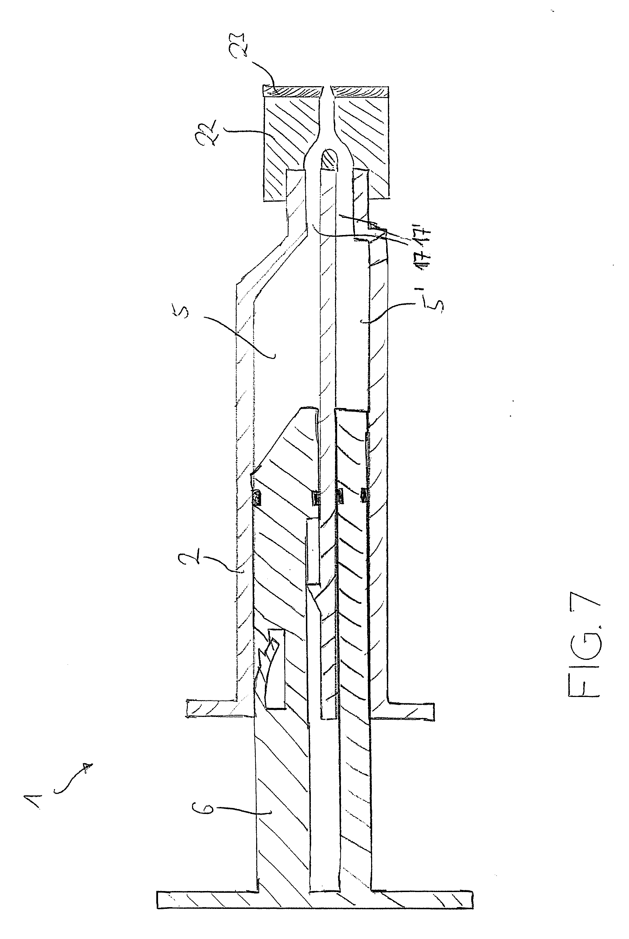

Finally, by at least partially inserting the pistons 7,7' into the chambers 5,5', the fluids are ejected through the openings 17,17' and (optionally) through the mixing assembly 22 and the spray assembly 23, as shown in Figure 7.

## Claims

1. A dispensing device (1), preferably a multi-chamber dispensing device, more preferably a two-chamber dispensing device, containing
- at least one chamber (5,5') for receiving a fluid (B,B'),
- a plunger unit (6) comprising at least one piston (7,7'), each piston (7,7') being arranged slidingly in one of the chambers (5,5'),
- locking means and counter-locking means, wherein the locking means and the counter-locking means can be brought into a locking position (L) in which a movement of the plunger unit (6) in one or both directions is substantially prevented,
**characterized by**
catching means and counter-catching means which can be brought into an engagement position (E) in which the locking means and the counter-locking means cannot be brought into the locking position (L).

2. The device (1) according to claim 1,
**characterized in that**
the locking means and the counter-locking means can be brought into a releasing position (R) in which a movement of the plunger unit (6) is possible in both directions.

3. The device (1) according to one of claims 1 to 2,
**characterized in that**
the catching means and the counter-catching means can be brought into a disengagement position (D) in which the locking means and the counter-locking means can be brought into the locking position (L).

4. The device (1) according to one of claims 1 to 3,
**characterized in that**
- the device has a front end (3) and a rear end (4), between which ends the at least one chamber (5,5') extends,
- in the locking position (L), a movement of the plunger unit (6) in the direction from the rear end (4) to the front end (5) is substantially prevented.

5. The device (1) according to claim 4,
**characterized in that**
- when the catching means and the counter-catching means are in the disengagement position (D) and the locking means and the counter-locking means are in the releasing position (R), the locking means and/or the counter-locking means are resiliently supported by a tensioned supporting element, in particular radially;
- when the catching means and the counter-catching means are in the disengagement position (D) and the locking means and the counter-locking means are in the locking position (L), the supporting element is less tensioned.

6. The device (1) according to one of claims 1 to 5,
**characterized by**
stopping means which are arranged such that
- a movement of the plunger unit (6) is limited in the direction from the front end (3) to the rear end (4) of the device (1);
- when the locking means and the counter-locking means are in the locking position (L), the plunger unit (6) is movable in the direction from the front end (3) to the rear end (4) by a pre-defined distance.

7. The device (1) according to one of claims 1 to 6,
**characterized in that**
the locking means comprise at least one radial protrusion (12), in particular at least one radially extending tooth.

8. The device (1) according to one of claims 1 to 7,
**characterized in that**
the plunger unit (6) contains the catching means and the counter-catching means.

9. The device (1) according to claim 8,
**characterized in that**
the plunger unit (6) comprises
- at least one retaining arm (13) containing a tip (14) forming the catching means and
- a retaining surface (15) forming the counter-catching means.

10. The device (1) according to claim 9,
**characterized in that**
the retaining arm (13) forms the supporting element.

11. The device (1) according to one of claims 1 to 10,
**characterized in that**
the plunger unit (6), in particular the retaining arm (13), contains the locking means.

12. Multi-chamber dispensing device (1), in particular dispensing device (1) according to one of claims 1 to 11, containing
- at least two chambers (5) for receiving a fluid (B,B'),
- a plunger unit (6) comprising at least two pistons (7), each piston (7) being arranged slidingly in one of the chambers (5),
wherein
- each chamber (5,5') has an end surface (16,16') having an opening (17,17'),
- at least one of the chambers (5,5') is designed such that
○ its cross-section is reduced in the direction of the respective opening (17,17') and
○ when the device (1) is tilted in a tilting direction (C) by a tilting angle (α) not exceeding a critical tilting angle (α_{crit}), all chambers (5,5') have essentially no dead volume,
- the openings (17,17') are arranged adjacent to one another,
**characterized in that**
a tip surface (18,18') of at least one piston (7,7') is essentially complementary to the end surface (16,16') of the respective chamber (5,5').

13. The device (1) according to claim 12,
**characterized in that**
the at least one surface (16,16'), in particular all end surfaces (16,16') are essentially planar and inclined by an angle with respect to a plane which is perpendicular to a longitudinal axis (A) of the device (1).

14. A kit (19) containing a device (1) according to one of claims 1 to 13 and at least one of the following components:
- a multiple connecting adapter (20), in particular a double connecting adapter, for directly or indirectly connecting openings (17,17') of each of the chambers (5,5') with a respective fluid reservoir (21) simultaneously;
- a mixing assembly (22) for mixing fluids (B,B') to be ejected from the chambers (5,5');
- a spray assembly (23) for spraying fluids (B,B') to be ejected from the chambers (5,5').

15. A method of operating a device (1) according to one of claims 3 to 13 or a kit (19) according to claim 14, wherein the method comprises the following steps:
i) providing at least one fluid reservoir (21,21'), at least one of the fluid reservoirs (21,21') containing a fluid (B,B');
ii) directly or indirectly connecting openings (17,17') of each chamber (5,5') of the device (1) with a respective fluid reservoir (21,21'), in particular by a multiple connecting adapter (20);
iii) at least partially retracting the piston or pistons (7,7') of the device (1) from the chamber or chambers (5,5');
iv) allowing the locking means and the counter-locking to obtain the locking position (L);
v) disconnecting the openings (17,17') from the respective fluid reservoirs (21,21');
vi) bringing the catching means and the counter-catching means into the disengagement position (D), in particular by a one-hand operation;
vii) at least partially inserting the pistons (7,7') into the chambers (5,5'), thereby ejecting the fluids (B,B'), in particular through a mixing assembly (22) and/or a spray assembly (23).
